# EUROPEAN PATENT SPECIFICATION

(11) **EP 3 936 575 B1**
(45) Date of publication and mention of the grant of the patent: **31.12.2025**
(21) Application number: 21182017.0
(22) Date of filing: 28.06.2021
(51) Int. Cl.: C09C 1/00, C09C 1/02, C09C 1/04, C09C 1/24, C09C 1/30, C09C 1/36, C09C 1/40, C09C 1/42, C09C 3/08, A61K 8/02, A61K 8/06, A61K 8/29, A61K 8/49, A61Q 19/00

(54) **SURFACE-TREATED INORGANIC PARTICLES, MANUFACTURING METHOD OF THE SAME, DISPERSION SOLUTION OF THE SAME, AND COSMETIC COMPOSITION INCLUDING THE SAME**
OBERFLÄCHENBEHANDELTE ANORGANISCHE TEILCHEN, HERSTELLUNGSVERFAHREN DAFÜR, DISPERSIONSLÖSUNG DAVON UND KOSMETISCHE ZUSAMMENSETZUNG DAMIT
PARTICULES INORGANIQUES TRAITÉES EN SURFACE, LEURS PROCÉDÉ DE FABRICATION, SOLUTION DE DISPERSION DE CELLES-CI ET COMPOSITION COSMÉTIQUE LES COMPRENANT

(30) Priority: 29.06.2020 KR 20200079409
(43) Date of publication of application: 12.01.2022
(73) Proprietor: Amorepacific Corporation, Seoul 04386 (KR)
(72) Inventor: SHIM, Jongwon, Seongnam-si, Gyeonggi-do (KR); YOU, Jaewon, Yongin-si, Gyeonggi-do (KR); KIM, Bomin, Nonsan-si, Chungcheongnam-do (KR); LEE, Seunghwan, Yongin-si, Gyeonggi-do (KR); BAEK, Heungsoo, Yongin-si, Gyeonggi-do (KR); PARK, Wonseok, Yongin-si, Gyeonggi-do (KR)
(74) Representative: Viering, Jentschura & Partner mbB Patent- und Rechtsanwälte

(56) References cited:
- US-A1- 2014 206 630
- KE XIANG ET AL: "Highly Augmented Antioxidant and Anticancer Effect of Biocompatible MIL-100(Fe)@SiO-Immobilized Green Tea Catechin", JOURNAL OF INORGANIC AND ORGANOMETALLIC POLYMERS AND MATERIALS, SPRINGER US, NEW YORK, vol. 30, no. 3, 8 July 2019 (2019-07-08), pages 935 - 942, XP037012362, ISSN: 1574-1443, [retrieved on 20190708], DOI: 10.1007/S10904-019-01257-2
- PATRICIA HORCAJADA ET AL: "Synthesis and catalytic properties of MIL-100(Fe), and iron(III) carboxylate with large pores", CHEMICAL COMMUNICATIONS, ROYAL SOCIETY OF CHEMISTRY, UK, vol. 2007, no. 27, 1 January 2007 (2007-01-01), pages 2820 - 2822, XP002652109, ISSN: 1359-7345, [retrieved on 20070515], DOI: 10.1039/B704325B
- XUERUI CHEN, ZHEQI SHI, RONGLIANG TONG, SHIPING DING, XU WANG, JIAN WU, QUNFANG LEI, WENJUN FANG: "Derivative of Epigallocatechin-3-gallatea Encapsulated in ZIF-8 withPolyethylene Glycol-Folic Acid Modification for Target and pHResponsiveDrug Release in Anticancer Research", ACS BIOMATER. SCI. ENG., vol. 2018, no. 4, 9 November 2018 (2018-11-09), pages 4183 - 4192, XP002804868, DOI: 10.1021/acsbiomaterials.8b00840

## Description

### CROSS-REFERENCE TO RELATED APPLICATION

This application claims priority to and the benefit of Korean Patent Application No. 10-2020-0079409 filed in the Korean Intellectual Property Office on June 29, 2020.

### BACKGROUND OF THE INVENTION

### (a) Field of the Invention

This disclosure relates to surface-treated inorganic particles, manufacturing methods of the same, dispersion solutions of the same, and cosmetic compositions including the inorganic particles or dispersion solutions.

### (b) Description of the Related Art

Inorganic particles, in particular, inorganic nanoparticles, have new characteristics not found in tens to hundreds of conventional micro-sized inorganic particles and often exhibit much improved characteristics and thus are being developed in various fields of semiconductors, displays, machines, cars, household goods, and the like.

These inorganic nanoparticles have representative characteristics that are classified into optical, physical/chemical, and magnetic characteristics and the like. In particular, as for very small-sized inorganic nanoparticles such as quantum dots, as the particle size is smaller, the surface atoms have a larger electron energy level difference. Color produced by absorbing light may be exhibited from movement of electrons between energy levels, and a difference in energy levels causes changes in wavelength bands and colors of the absorbed light, so color changes according to sizes of quantum dots. The nano-sized inorganic nanoparticles are applied to a display having a high color reproduction rate, a medical industry such as analysis, diagnosis, and the like, and an electronics industry.

In addition, since the nano-sized inorganic particles have a large surface area per unit volume, the number of atoms on the surfaces of the particles relative to a total number of atoms is increased. In other words, the inorganic nanoparticles exist in an unstable state with increased surface energy. In this way, since the unstable particles easily overcome an energy barrier for a reaction by receiving a small amount of energy, they are used as a catalyst in various reactions due to the excellent reactivity. For example, titanium dioxide having a size of less than or equal to about 20 nm has effects of sterilization, cleaning, anti-fog, and the like by receiving weak ultraviolet (UV) rays from fluorescent lamps. In addition, since magnetic properties of the inorganic nanoparticles are also increased, as intensity of magnetic fields around the atoms is increased, the inorganic nanoparticles are applied as a medical material such as for MRI (magnetic resonance imaging).

However, despite the high potential and growth of this inorganic nanoparticle material, its industrial applications are still underdeveloped. The main reasons may be its chemical instability due to high surface energy of inorganic nanoparticle powder, agglomeration, a costly manufacturing process, lack of safety assessment research on a human body and the environment, and the like. Among these, the most common problem is that the inorganic nanoparticles have low dispersion stability and thus may be easily naturally ignited and oxidized due to the high surface energy, and in order to reduce the surface energy, the inorganic nanoparticles are dispersed in a solvent to prevent agglomeration among the particles but are gelled due to sharply increased viscosity at a particular concentration, and even after being temporarily dispersed by applying high energy thereto, the inorganic nanoparticles are rapidly reagglomerated and phase-separated and thus are nonuniformly coated and fail in fully functioning.

For example, titanium dioxide (TiO₂) powder, an inorganic metal oxide, is widely used as a white pigment for various paints, for example, a raw material of an ultraviolet (UV) blocking agent for cosmetics absorbing and reflecting ultraviolet (UV), a semiconductor, and a food additive, but is hardly dispersed in various solvents at a high concentration and also has the common problems of nano-oxide particles such as rapid agglomeration and phase separation. **In** addition, reactive oxygen species (ROS) radicals generated by strong photocatalystic activity of titanium dioxide are known to cause discoloration and oxidization of other raw materials in formulation of cosmetics, medicine, food, and the like, and also attack cell membranes and cell nuclei of living bodies and cause DNA damage, cancer, dementia, and aging.

A common method for solving this problem is to use a dispersion stabilizer such as a surfactant and the like along with the inorganic metal oxide (inorganic particles).

However, even if the dispersion stabilizer is used, dispersion stability of the inorganic particles is not improved to a satisfactory level, and the disperse stabilizer deteriorates or removes antioxidant ability (a radical removal rate) of the inorganic particles, and accordingly, when the inorganic particles are used along with other easily discolored raw materials, the problem of significantly deteriorating sensory properties and efficacy of final products due to the discoloration of these other raw materials is not still solved.

On the other hand, polyphenols include tannin that is abundant in the stems of plants or the peel of fruits, catechins contained in a large amount in green tea, resveratrol in grapes, quercetin in a large amount in apples and onions and the like, and is a beneficial substance that can delay aging caused by cell damage in the human body through a strong antioxidant action. However, polyphenols have excellent astringent action, positive skin action, protective action, anti-mutation action, hemostasis action, detoxification action, antioxidant action, sebum secretion suppression action, and the like, but also have drawbacks of high instability and easy discoloration by heat or oxygen. Green tea or apple juice is discolored when left, which is caused by the polyphenols.

Accordingly, there are attempts to apply the polyphenols, particularly, the catechins, to a cosmetic composition and thus realize excellent efficacy of the catechins, but since this discoloration problem is not still solved, efforts to develop a cosmetic composition having no discoloration problem by using the catechins are still being made to this day.

Tea catechins in nature largely include 8 types of components. Green tea includes greater than or equal to 10 %, greater than or equal to 11 %, greater than or equal to 12 %, greater than or equal to 13 %, greater than or equal to 14 %, less than or equal to 15 %, less than or equal to 14 %, less than or equal to 13 %, less than or equal to 12 %, less than or equal to 11 % of the tea catechins based on a total dry weight of green tea leaves, and epigallocatechin gallate (EGCG) is greater than or equal to about 50 %, greater than or equal to about 51 %, greater than or equal to about 52 %, greater than or equal to about 53 %, greater than or equal to about 54 %, greater than or equal to about 55 %, greater than or equal to about 56 %, greater than or equal to about 57 %, greater than or equal to about 58 %, greater than or equal to about 59 % of the tea catechins. The epigallocatechin gallate (EGCG) is less than or equal to about 60 %, less than or equal to about 59 %, less than or equal to about 58 %, less than or equal to about 57 %, less than or equal to about 56 %, less than or equal to about 55 %, less than or equal to about 54 %, less than or equal to about 53 %, less than or equal to about 52 %, less than or equal to about 51 % of the tea catechins. Gallocatechin gallate (GCG) is a #2 carbon isomer of epigallocatechin gallate (EGCG), and is a low content catechin that is only greater than or equal to about 0.8 %, greater than or equal to about 0.9 %, greater than or equal to about 1.0 %, greater than or equal to about 1.1 %, greater than or equal to about 1.2 %, greater than or equal to about 1.3 %, greater than or equal to about 1.4 %, less than or equal to about 1.5 %, less than or equal to about 1.4 %, less than or equal to about 1.3 %, less than or equal to about 1.2 %, less than or equal to about 1.1 %, less than or equal to about 1.0 %, less than or equal to about 0.9 % of catechins present in green tea.

The catechins in green tea are representatively epicatechin [(-)EC], epigallocatechin [(-)EGC], epicatechin gallate [(-)ECG], and epigallocatechin gallate [(-)EGCG], and these catechins have representative activity covering from antioxidant effects of green tea, which has already been reported, to a cholesterol absorption inhibitory action, triglyceride suppression, antivirus action, anti-tumor action, anti-cancer action, skin beautification action, obesity suppression action, mutagenic suppression action, etc. Recently, these catechins have been respectively converted into non-epithetic catechins, that is, catechin [(-)C], gallocatechin [(-)GC], catechin gallate [(-)CG], and gallocatechin gallate [(-)GCG] during a pasteurization process of green tea beverages. In addition, when hot water is poured into green tea leaves, epithetic catechin of the green tea leaves is converted into catechin epimer, which is a non-epithetic catechin. These isomers exhibit equivalent antioxidant efficacy to that of each isomer (Xu JZ et al., British Journal of Nutrition, 91, 873-881, 2004; Unno T et al., J. Sci. Food Agri., 80, 601-606, 2000). The catechin gallate and the gallocatechin gallate exhibit more excellent cholesterol absorption inhibitory action than that of their isomers (Ikeda I et al., J. Agric. Food Chem., 51, 7303-7307, 2003). As for anti-allergic efficacy, the epigallocatechin gallate is also superior to that of its isomer, gallocatechin gallate (Nagai H et al., J. Sci. Food Agri., 85, 1606-1612, 2005), and the gallocatechin gallate activates PPAR (Peroxisome Proliferator-Activated Receptor)-alpha, and promotes expression of filaggrin, a skin-moisturizing factor, and thus has excellent skin drying prevention and skin moisturizing effects (Korea Patent Application No. 2005-0086821). In addition, non-epithetic catechin present in green tea beverages may not only stabilize epithetic catechin but also has more excellent effects on taste. In this way, as the possibility of industrial utility of the non-epithetic catechin such as gallocatechin gallate and the like has been revealed, the non-epithetic catechin has undergone more attempts to use it but is included in a very small content in green tea, and in addition, since the conversion of catechin epimers, to date, necessarily requires a high temperature, pH control, and the like, it is impossible to mass-produce the non-epithetic catechin such as the gallocatechin gallate and the like through extraction, separation, and purification from green tea leaves. Further metal-organic frameworks are known from K. Xiang et al, Journal of inorganic and organometallic polymers and materials, Vol. 30, No 3, 935-942, 2019 & P. Horcajada et al., Chemical communications, Royal society of chemistry, Vol. 2007, No 27, 2820-2822; X. Chen et al, ACS Biomater. Sci. Eng, Vol 2018, No 4, 4183-4192 and US 2017/206630 A.

### SUMMARY OF THE INVENTION

An embodiment provides surface-treated inorganic particles including catechins capable of inhibiting discoloration for a long time without losing the antioxidant ability of catechins.

Another embodiment provides a method of manufacturing the surface-treated inorganic particles.

Another embodiment provides a dispersion solution in which the surface-treated inorganic particles are dispersed.

Another embodiment provides a cosmetic composition including the surface-treated inorganic particles or the dispersion solution.

According to an embodiment, surface-treated inorganic particles include inorganic particles and a metal-organic framework bound to the surface of the inorganic particles, wherein catechins form a skeleton of the metal-organic framework.

The catechins may include epicatechin, epicatechin gallate, epigallocatechin, epigallocatechin gallate, catechin, catechin gallate, gallocatechin, gallocatechin gallate, or a combination thereof.

The metal constituting the metal-organic framework may include iron, magnesium, zinc, copper, manganese, titanium, molybdenum, cerium, zirconium, barium, aluminum, calcium, yttrium, silver, gold, or a combination thereof.

The inorganic particles may include titanium dioxide, zinc oxide, iron oxide, copper oxide, aluminum oxide, zirconium oxide, cerium oxide, barium oxide, silica, mica, talc, sericite, calamine, or a combination thereof.

The inorganic particles may have a particle diameter of about 10 nm to about 100,000 nm.

According to another embodiment, a method of manufacturing surface-treated inorganic particles includes: coordinating catechins to the surface of inorganic particles; polymerizing the coordinated catechins with metal ions; and purifying the surface-treated inorganic particles.

The coordinating of the catechins to the surface of inorganic particles may include adding the inorganic particles to an aqueous solution, dispersing the resultant by using ultrasonic waves or the like, adding the catechins thereto, and stirring the resultant.

Herein, a content of the catechins may be less than or equal to about 20 parts by weight relative to 100 parts by weight of the inorganic particles to be added.

The adding of the inorganic particles to the aqueous solution, dispersing of the resultant by using ultrasonic waves or the like, adding of the catechins thereto, and stirring of the resultant may be performed for about 1 minute to about 60 minutes at a temperature of about 10 °C to about 30 °C under a pH condition of about 4 to about 9.

The polymerizing of the coordinated catechins with metal ions may be performed by injecting the metal ions or the cluster thereof to an aqueous solution in which inorganic particles having coordination bonds with catechins on the surface are dispersed.

The injecting of the metal ions or the cluster thereof to an aqueous solution in which inorganic particles having coordination bonds with catechins on the surface are dispersed may include stirring the solution for about 1 minute to about 6 hours at about 10 °C to about 100 °C after injecting the metal ions or the cluster thereof.

The purifying of the surface-treated inorganic particles may include repeating the removal of a supernatant by filtration or centrifugation using a filter having nanopores or micropores.

The catechins, metal ions, inorganic particles, and the like may be as described above.

According to another embodiment, a dispersion solution in which the surface-treated inorganic particles are dispersed is provided.

A solid content in the dispersion solution may be about 0.1 wt% to about 70 wt% relative to the total amount of the dispersion solution.

According to another embodiment, a cosmetic composition including the surface-treated inorganic particles or the dispersion solution is provided.

Using the surface-treated inorganic particles according to an embodiment, it is possible to provide a cosmetic composition that has improved antioxidant ability and is not discolored even if left for a long time.

### BRIEF DESCRIPTION OF THE DRAWINGS

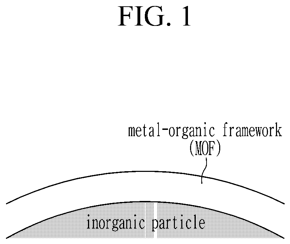

FIG. 1 is a schematic view showing a cross-section of a surface-treated inorganic particle (Preparation Example 1) according to an embodiment.

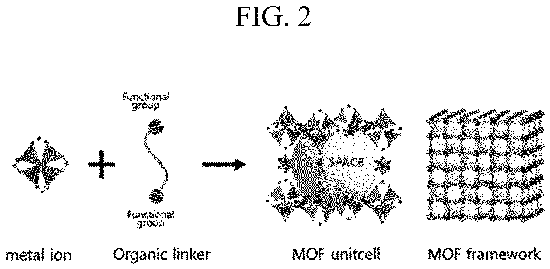

FIG. 2 is a schematic view showing a process of forming a metal-organic framework by a metal ion and an organic linker.
FIGS. 3 and 5 are transmission scanning electron micrographs before surface-treatment of titanium dioxide nanoparticles, respectively.
FIGS. 4 and 6 are transmission scanning electron micrographs of the titanium dioxide nanoparticles which are independently surface-treated.
FIG. 7 is a graph showing the antioxidant ability of catechins bound to titanium dioxide nanoparticles (coordination in the form of MOF) and catechins not bound to titanium dioxide nanoparticles.
FIG. 8 is a photograph taken after leaving the cosmetic composition according to Example 1 at room temperature for 5 weeks.
FIG. 9 is a photograph taken after leaving the cosmetic composition according to Example 1 at 40 °C for 5 weeks.
FIG. 10 is a photograph taken after leaving the cosmetic composition according to Comparative Example 1 at room temperature for 5 weeks.
FIG. 11 is a photograph taken after leaving the cosmetic composition according to Comparative Example 1 at 40 °C for 5 weeks.

### DETAILED DESCRIPTION OF THE EMBODIMENTS

Hereinafter, embodiments of one aspect of the present disclosure will be described in detail so that those skilled in the art to which one aspect of the present disclosure pertains could easily practice it. However, this disclosure may be embodied in many different forms and is not to be construed as limited to the example embodiments set forth herein.

As used herein, it will be understood that when an element such as a layer, film, region, or substrate is referred to as being "on" another element, it may be directly on the other element or intervening elements may also be present. In contrast, when an element is referred to as being "directly on" another element, there are no intervening elements present.

As used herein, when a definition is not otherwise provided, the term "combination" refers to mixing or copolymerization. Also, "copolymerization" is block copolymerization or random copolymerization, and "copolymer" is a block copolymer or a random copolymer.

As used herein, when a definition is not otherwise provided, catechins refer to 8 types of catechins, and the 8 types of catechins are composed of 4 types of epithetic catechins and 4 types of non-epithetic catechins. The 4 types of epithetic catechins may include epigallocatechin, epicatechin, epigallocatechin gallate, and epicatechin 3-O-gallate, and other 4 types of non-epithetic catechins constituting the 8 types of catechins may include gallocatechin, catechin, gallocatechin gallate, and catechin gallate.

Hereinafter, the surface-treated inorganic particles according to an embodiment are described.

An embodiment provides surface-treated inorganic particles that include inorganic particles and a metal-organic framework bound to the surface of the inorganic particles, wherein catechins form a skeleton of the metal-organic framework. More specifically, in an embodiment, the metal-organic framework is coordinated to a metal atom on the surface of the inorganic metal oxide particles to surface-treat the inorganic metal oxides, wherein catechins form the skeleton of the metal-organic framework.

A metal-organic framework (MOF) is an organic/inorganic hybrid (coordination compound) having a 1-dimensional, 2-dimensional, or 3-dimensional structure in which metal ions or a cluster of the metal ions form coordination bonds with organic molecules. In general, a metal-organic framework (MOF) may be formed by the interaction of catechins and metal ions, and in one aspect of the present disclosure, the metal-organic framework (MOF) is formed on the surface of inorganic particles using a non-ordinary method in which coordination bonds between inorganic particles and catechins are first induced, and then a metal-organic framework (MOF) is formed on the surface of the inorganic particles. That is, the metal-organic framework (MOF) according to an embodiment is formed by interacting catechins with both inorganic particles and metal ions (or clusters thereof).

The coordination compound is a material produced by bonding metal ions or clusters thereof with other molecules having neutral or negative charges, and as for MOF having coordinatively unsaturated sites (CUS), an inorganic material connector itself includes a functional group and also exhibits semiconductor characteristics. MOF may be used as a basic material for activating a catalyst or generating an adsorption center in addition to inherent characteristics of CUS. The more well-known application field of MOF is to use much higher porosity than that of zeolite by internally designing hollow spaces of MOF nanopores in various methods to store carbon dioxide, the main cause of air pollution, or hydrogen, a raw material of a fuel cell, inside the MOF nanopores having appropriate synthesis or functional group or to use various catalysts. In addition, the MOF pores may be designed to be contracted or transformed by light or heat and thus used for storing or emitting a dye or a drug.

On the other hand, catechins, one type of polyphenols, are a beneficial material having strong antioxidant ability and thus delay aging by cell damage, as described above, and chemically have a molecular structure consisting of at least two phenol structures substituted with at least two hydroxy groups (a benzene ring substituted with at least two hydroxy groups) and thus easily form a coordination bond, and accordingly, the present inventors developed a cosmetic composition having excellent antioxidant ability and suppressed from discoloration even if left for a long time by bonding catechins with MOF and thus completed one aspect of the present disclosure after much trial and error over a long period of time. In general, research on application of the pores inside the metal-organic framework (MOF) has been actively made, and based on this research, a method of supporting catechins inside the pores has been introduced. However, since the method simply supports the catechins in the metal-organic framework but realizes no effect of stabilizing unstable catechins, an organic reducing agent and the like playing a role of stabilizing catechins is mostly used therewith. However, even though the organic reducing agent and the like are additionally used, the stabilization effect is limited, and accordingly, the discoloration of catechins may not be effectively suppressed for a long time.

The present inventors, as a result of many experiments and much research, found that when the catechins are not supported in the metal-organic framework but are formed into a skeleton of the metal-organic framework, the catechins may be coated (coordinated) on the surfaces of inorganic particles, specifically, on the surfaces of inorganic metal oxides, and thus easily form a hydrophilic coating film thereon, and accordingly, prevent strong agglomeration of the inorganic metal oxides and stably disperse the inorganic metal oxides in an aqueous phase at a high concentration and simultaneously strongly suppress the discoloration of the catechins during the long term storage, and resultantly, maintain excellent appearance and stable antioxidant performance.

On the other hand, in general, catechins have a coordination bond with inorganic particles, and specifically, inorganic metal oxides, and herein, the catechins have a functional group capable of having a coordination bond with a metal. Accordingly, the catechins may be used to easily form the metal-organic framework, which is one type of coordination compound and has the catechins as a skeleton, on the surfaces of the inorganic metal oxide particles mainly used in a cosmetic composition.

As aforementioned, the metal-organic framework has nanopores and much higher porosity than that of zeolite and thus is used for storing hydrogen in the nanopores or as a catalyst or a basis for generating adsorption centers through appropriate synthesis or applying a functional group thereto, and accordingly, has been studied not only for a reactivity catalyst, a fuel cell, or the like, but also as a porous nanocarrier for drug transport or a thin film for controlling a drug permeation rate and antimicrobial and hydrophilic surface-treatment technology by using tannic acid, a polyphenol component easily forming a coordination bond. Accordingly, the present inventors confirmed that the metal-organic framework may be synthesized by using a natural material (catechins and the like) having a coordination bond in an aqueous solution and a small amount of metal ions or a cluster thereof, and then completed one aspect of the present disclosure by first mixing a catechin component, polyphenols, with inorganic metal oxide particles, forming a coordination bond on the surface of the inorganic metal oxide particles, and mixing polyvalent metal ions therewith to raise a metal-organic framework thin film having a skeleton formed of the catechin component, and then, by examining dispersion stability, light absorption characteristics, antioxidant activity, and discoloration stability of the formed thin film.

For example, the catechins may include 8 types of catechins, specifically epicatechin, epicatechin gallate, epigallocatechin, epigallocatechin gallate, catechin, catechin gallate, gallocatechin, gallocatechin gallate, or a combination thereof, and more specifically, the catechins may include epicatechin, epigallocatechin gallate, catechin, gallocatechin gallate, or a combination thereof.

For example, the metal constituting the metal-organic framework may include iron, magnesium, zinc, copper, manganese, titanium, molybdenum, cerium, zirconium, barium, aluminum, calcium, yttrium, silver, gold, or a combination thereof, but is not necessarily limited thereto.

For example, the metal ion includes an iron ion, a magnesium ion, a zinc ion, a copper ion, a manganese ion, a titanium ion, a molybdenum ion, a cerium ion, a zirconium ion, a barium ion, an aluminum ion, a calcium ion, a yttrium ion, a silver ion, a gold ion, or a combination thereof, but is not necessarily limited thereto.

For example, the inorganic particles may include titanium dioxide, zinc oxide, iron oxide, copper oxide, aluminum oxide, zirconium oxide, cerium oxide, barium oxide, silica, mica, talc, sericite, calamine, or a combination thereof, but are not limited to thereto.

For example, the inorganic particles may have a particle diameter of greater than or equal to about 10 nm, greater than or equal to about 20 nm, greater than or equal to about 30 nm, greater than or equal to about 40 nm, greater than or equal to about 50 nm, greater than or equal to about 60 nm, greater than or equal to about 70 nm, greater than or equal to about 80 nm, greater than or equal to about 90 nm, greater than or equal to about 100 nm, greater than or equal to about 200 nm, greater than or equal to about 300 nm, greater than or equal to about 400 nm, greater than or equal to about 500 nm, greater than or equal to about 600 nm, greater than or equal to about 700 nm, greater than or equal to about 800 nm, greater than or equal to about 900 nm, greater than or equal to about 1000 nm.

For example, the inorganic particles may have a particle diameter of less than or equal to about 100,000 nm, less than or equal to about 90,000 nm, less than or equal to about 80,000 nm, less than or equal to about 70,000 nm, less than or equal to about 60,000 nm, less than or equal to about 50,000 nm. That is, the inorganic particles may have excellent dispersion stability in an aqueous phase in both nanometer size and micrometer size.

Another embodiment provides a method of manufacturing the surface-treated inorganic particles, and the manufacturing method includes: coordinating catechins to the surface of inorganic particles; polymerizing the coordinated catechins with metal ions; and purifying the surface-treated inorganic particles.

For example, the coordinating of catechins to the surface of inorganic particles may include adding inorganic particles to an aqueous solution, dispersing them using ultrasonic waves, etc., and adding the catechins followed by stirring the resultant. The polymerizing of the coordinated catechins with metal ions may include injecting the metal ions or the cluster thereof to an aqueous solution in which inorganic particles having coordination bonds with catechins on the surface are dispersed.

All materials used in the method of manufacturing the surface-treated inorganic particles are environmentally friendly. After mixing catechins in deionized water at a certain ratio, inorganic metal oxide particles such as titanium dioxide are added to this solution, and ultrasonic waves are applied to homogeneously disperse it in a solvent and to induce coordination bonds, and the resultant is stirred again to inject the metal ions or metal ion cluster at room temperature to proceed with polymerization of the organic/inorganic hybrid framework. Herein, sizes of the inorganic metal oxide particles used are not limited, but inorganic metal oxide particles having a size of about 10 nm to about 100,000 nm may be used, and depending on the sizes of the particles, a content of injected catechins may be within about 20 % based on a weight of the inorganic metal oxide particles. That is, the content of the catechins may be less than or equal to about 20 parts by weight, for example about 1 part by weight to about 20 parts by weight, about 1 part by weight to about 10 parts by weight, about 1 part by weight to about 4 parts by weight, about 1 part by weight to about 2 parts by weight, about 2 part by weight to about 20 parts by weight, about 4 parts by weight to about 20 parts by weight, or about 10 parts by weight to about 20 parts by weight based on 100 parts by weight of the injected inorganic particles. When the content of the catechins is less than 1 part by weight based on 100 parts by weight of the injected inorganic particles, the content of the catechins is too small to form a skeleton of a metal-organic structure, while when the content of the catechins is greater than about 20 parts by weight based on 100 parts by weight, the difference in effect may be insignificant compared to the case of 1 to 20 parts by weight, and thus may be uneconomical. The adding of the inorganic particles to the aqueous solution, dispersing them using ultrasonic waves, etc., and adding the catechins followed by stirring the resultant may be performed for greater than or equal to about 1 minute, greater than or equal to about 5 minutes, greater than or equal to about 10 minutes, greater than or equal to about 20 minutes, greater than or equal to about 30 minutes, greater than or equal to about 40 minutes, greater than or equal to about 50 minutes, less than or equal to about 60 minutes, less than or equal to about 50 minutes, less than or equal to about 40 minutes, less than or equal to about 30 minutes, less than or equal to about 20 minutes, less than or equal to about 10 minutes, less than or equal to about 5 minutes at a temperature of greater than or equal to about 10 °C, greater than or equal to about 15 °C, greater than or equal to about 20 °C, greater than or equal to about 25 °C, less than or equal to about 30 °C, less than or equal to about 25 °C, less than or equal to about 20 °C, less than or equal to about 15 °C under a pH condition of greater than or equal to about 4, greater than or equal to about 5, greater than or equal to about 6, greater than or equal to about 7, greater than or equal to about 8, less than or equal to about 9, less than or equal to about 8, less than or equal to about 7, less than or equal to about 6, less than or equal to about 5. In addition, the injecting of the metal ions or the cluster thereof to the aqueous solution in which inorganic particles having coordination bonds with catechins on the surface are dispersed may include stirring the solution for greater than or equal to about 1 minute, greater than or equal to about 5 minutes, greater than or equal to about 10 minutes, greater than or equal to about 20 minutes, greater than or equal to about 30 minutes, greater than or equal to about 40 minutes, greater than or equal to about 50 minutes, greater than or equal to about 1 hour, greater than or equal to about 2 hours, greater than or equal to about 3 hours, greater than or equal to about 4 hours, greater than or equal to about 5 hours, less than or equal to about 6 hours, less than or equal to about 5 hours, less than or equal to about 4 hours, less than or equal to about 3 hours, less than or equal to about 2 hours, less than or equal to about 1 hour, less than or equal to about 50 minutes, less than or equal to about 40 minutes, less than or equal to about 30 minutes, less than or equal to about 20 minutes, less than or equal to about 10 minutes, less than or equal to about 5 minutes at greater than or equal to about 10 °C, greater than or equal to about 20 °C, greater than or equal to about 30 °C, greater than or equal to about 40 °C, greater than or equal to about 50 °C, greater than or equal to about 60 °C, greater than or equal to about 70 °C, greater than or equal to about 80 °C, greater than or equal to about 90 °C, less than or equal to about 100 °C, less than or equal to about 90 °C, less than or equal to about 80 °C, less than or equal to about 70 °C, less than or equal to about 60 °C, less than or equal to about 50 °C, less than or equal to about 40 °C, less than or equal to about 30 °C, less than or equal to about 20 °C after injecting the metal ions or the cluster thereof. Herein, a content of the metal ions or cluster thereof may be less than or equal to about 1 part by weight, for example about 0.05 parts by weight to about 1 part by weight, about 0.05 parts by weight to about 0.5 parts by weight, about 0.05 parts by weight to about 0.2 parts by weight, about 0.05 parts by weight to about 0.1 parts by weight, about 0.1 parts by weight to about 1 part by weight, about 0.2 parts by weight to about 1 part by weight, or about 0.5 parts by weight to about 1 part by weight based on 100 parts by weight of the injected inorganic particles. The reaction speed may be controlled by changing a temperature and pH, and when the metal-organic framework is completely synthesized, the coated inorganic particles are repeatedly washed at least twice with a filter having nano-sized or micro-sized pores or centrifuged to remove supernatants and dispersed (in water) again to obtain a dispersion solution, or the washed particles are dried and pulverized to obtain a powder.

A thickness of the "metal-organic framework thin film in which catechins form a skeleton" uniformly formed on the metal oxide nanoparticle according to the method of one aspect of the present disclosure may be adjusted according to a ratio between the inorganic metal oxide particles and the catechins used for the synthesis, and accordingly, the inorganic metal oxide particles having the metal-organic framework on the surfaces, even though mixed in an aqueous phase at a high weight ratio, are not agglomerated but are stably dispersed at low viscosity, and when stored for a long time at room temperature and a high temperature, exhibit no discoloration of the catechins. In general, particular coordination bond colors appear in various spectrum regions depending on types of inorganic metal oxide nanoparticles and types of catechins, and when used according to the composition of one aspect of the present disclosure, the inorganic metal oxide particles coordinated with the "metal-organic framework in which catechins form a skeleton" on the surfaces exhibit a coordination bond color changing from initially light yellowish brown to white through the aging process and no color change by oxidization of catechins molecules during the long-term storage.

The catechins, metal ions, and inorganic particles used in the method for manufacturing the surface-treated inorganic particles are the same as those described in the description of the aforementioned surface-treated inorganic particles, unless otherwise specified.

Another embodiment provides a dispersion solution in which the surface-treated inorganic particles are dispersed.

A solid content in the dispersion solution may be greater than or equal to 0.1 wt%, greater than or equal to 0.5 wt%, greater than or equal to 1 wt%, greater than or equal to 5 wt%, greater than or equal to 10 wt%, greater than or equal to 20 wt%, greater than or equal to 30 wt%, greater than or equal to 40 wt%, greater than or equal to 50 wt%, greater than or equal to 60 wt%, less than or equal to 70 wt%, less than or equal to 60 wt%, less than or equal to 50 wt%, less than or equal to 40 wt%, less than or equal to 30 wt%, less than or equal to 20 wt%, less than or equal to 10 wt%, less than or equal to 5 wt%, less than or equal to 1 wt%, less than or equal to 0.5 wt% relative to a total amount of the dispersion solution.

Another embodiment provides a cosmetic composition including the surface-treated inorganic particles or the dispersion solution.

Since both the dispersion solution and the cosmetic composition include inorganic metal oxide particles coated with the "metal-organic framework in which catechins form a skeleton," they may prevent a browning phenomenon that occurs in the formulation even when left for a long time while maintaining excellent antioxidant properties of catechins and thus a product itself may be greatly improved compared with products containing conventional catechins.

Cosmetic formulations to which the surface-treated inorganic metal oxide particles are applied may include lotions, nourishing creams, nourishing lotions, eye creams, essences, cleansing creams, cleansing lotions, packs, body lotions, body creams, body essences, makeup bases, foundations, ointments, gels, creams, patches, and the like. A blending component included in addition to the surface-treated inorganic metal oxide particles may include a fat or oil component, a moisturizing agent, an emollient agent, a surfactant, an organic or inorganic pigment, an organic powder, an ultraviolet absorber, preservatives such as phenoxyethanol or 1,2-hexanediol, antiseptics, antioxidants, plant extracts, pH adjusters, alcohols, pigments, fragrances such as artificial flavors, blood circulation accelerators, coolants, limiting agents, purified water, and the like. In addition, the compounding components which may be added are not limited thereto, and any of the above components may be compounded within a range not impairing purposes and effects of one aspect of the present disclosure.

When the formulations of one aspect of the present disclosure are solutions or emulsions, a solvent, a solubilizer, or an emulsifier is used as a carrier component, for example water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, a fatty acid ester of sorbitan, and the like.

When the formulations of one aspect of the present disclosure are suspensions, liquid diluents such as water, ethanol, or propylene glycol as carrier components, suspension agents such as ethoxylated isostearyl alcohol, polyoxyethylene sorbitol esters, and polyoxyethylene sorbitan esters, microcrystalline cellulose, aluminum metahydroxide, bentonite, agar, or tragacanth, and the like may be used.

When the formulations of one aspect of the present disclosure are pastes, creams, or gels, animal oil, vegetable oil, wax, paraffin, starch, tragacanth, cellulose derivatives, polyethylene glycol, silicone, bentonite, silica, talc, zinc oxide, and the like may be used as a carrier component.

When the formulations of one aspect of the present disclosure are powders or sprays, lactose, talc, silica, aluminum hydroxide, calcium silicate, or polyamide powders may be used as a carrier component. **In** particular, in the case of sprays, propellants such as chlorofluorohydrocarbon, propane/butane, or dimethyl ether may be additionally included.

The composition including the surface-treated inorganic particles or a dispersion solution thereof according to an embodiment may be used as a cosmetic composition as described above, as well as an external preparation for skin.

A formulation of the external preparation for skin is not limited thereto, and examples thereof include a liquid coating agent, a spray agent, a lotion agent, a gel agent, a paste agent, an ointment agent, an aerosol, a powder agent, and a transdermal absorber.

As the pharmaceutically acceptable carrier in the external preparation for skin, depending on its formulation, hydrocarbons such as petrolatum, liquid paraffin, gelled hydrocarbons (plastic base); animal and vegetable oils such as medium-chain fatty acid triglyceride, pork fat, hard fat, and cacao butter; higher fatty acid alcohols and fatty acids and esters thereof such as cetanol, stearyl alcohol, stearic acid, and isopropyl palmitate; water-soluble bases such as polyethylene glycol, 1,3-butylene glycol, glycerol, gelatin, white sugar, and sugar alcohols; emulsifiers such as glycerin fatty acid ester, polyoxyl stearate, and polyoxyethylene cured castor oil; adhesives such as acrylic acid esters and sodium alginate; propellants such as liquefied petroleum gas and carbon dioxide; and antiseptics such as paraoxybenzoic acid esters. Further, in addition to these, stabilizers, fragrances, colorants, pH adjusters, diluents, surfactants, preservatives, antioxidants, and the like may be formulated as necessary. The use of the external preparation for skin is desirably applied to aged cells by a conventional method.

In addition, the external preparation for skin may be adhered and used on a solid support such as a wound peeling cover of a conventional bandage. Adhesion may be achieved by saturating the composition according to an embodiment on a solid support followed by dehydration. For example, the solid support is first coated with an adhesive to improve adhesion of the solid support to the composition according to an embodiment. Examples of the adhesive may be polyacrylate, cyanoacrylate, and the like. This type of formulation is easily commercially available, for example, a bandage with a non-adhesive wound peeling cover in the form of a perforated plastic film (Smith & Nephew Ltd.); a thin strip (Johnson & Johnson Inc.), a patch, a spot, a plastic strip-shaped band-aid; Curity Curad Ouchless (Colgate-Palmolive Co. (Kendall)); and a Stik-Tite elastic strip (American White Cross Laboratories Inc.).

Advantages and features of one aspect of the present disclosure and methods for achieving them will be apparent with reference to the examples described below in detail. One aspect of the present disclosure will now be described in detail with reference to examples. However, these examples are specifically provided for describing one aspect of the present disclosure, and the range of one aspect of the present disclosure is not limited to these examples.

### (Preparation Examples)

### Preparation of Titanium Dioxide Particle Surface-treated with Metal-Organic Framework

### (Preparation Example 1)

Composite nanoparticles were synthesized by using titanium dioxide nanoparticles as seeds and coating epigallocatechin gallate (EGCG) as one of catechins on the surface of the titanium dioxide. First, the TiO₂ nanoparticles were added along with EGCG in a composition shown in Table 1 to an aqueous phase under a weak base condition of pH 8, and ultrasonic waves were applied thereto at room temperature for about 5 minutes to induce coordination bonds on the surfaces. Then, FeCl₃ as metal ions was mixed therewith, and then stirred at 30 °C for 30 minutes to form a metal-organic framework (MOF). This prepared EGCG-MOF-TiO₂ dispersion solution was filtered by using an aluminum oxide filter having pores of less than or equal to 200 nm and a powder obtained therefrom was redispersed in purified water, which were repeated twice for purification.

**(Table 1)**

| (unit: parts by weight) | | | |
|---|---|---|---|
| | TiO₂ | EGCG | FeCl₃ |
| Composition | 100 | 10 | 0.5 |

### (Preparation Example2)

Composite nanoparticles were synthesized according to the same method as Preparation Example 1, except that zinc oxide was used instead of the titanium dioxide as seeds.

### (Preparation Example 3)

Composite nanoparticles were synthesized according to the same method as Preparation Example 1, except that gallocatechin gallate (GCG) was used instead of EGCG.

### Preparation of Cosmetic Compositions: Examples 1 to 3 and Comparative Example 1

Cosmetic compositions of W/O emulsion formulation were prepared to have the compositions shown in Table 2 in a common method.

Specifically, W/O emulsions according to Examples 1 to 3 and Comparative Example 1 were prepared by heating and uniformly mixing oil-phase components and powder components at 75 °C according to each composition shown in Table 2, and then uniformly mixing them with aqueous-phase components, while stirring at the same temperature of 75 °C.

**(Table 2)**

| (unit: wt%) | | | | | |
|---|---|---|---|---|---|
| | Components | Ex. 1 | Ex. 2 | Ex. 3 | Comp. Ex. 1 |
| Oil-phase component | Butylene glycol dicaprylate/dicaprate | 6 | 6 | 6 | 6 |
| | Oil-phase component triisononanoin | 4 | 4 | 4 | 4 |
| | Oil-phase component octyl methoxycinnamate | 5 | 5 | 5 | 5 |
| | Oil-phase component cyclopentasiloxane/cyclohex asiloxane | 15 | 15 | 15 | 15 |
| | Oil-phase component tridecyl trimellitate | 3 | 3 | 3 | 3 |
| | Oil-phase component | 2 | 2 | 2 | 2 |
| | dimethicone/vinyl dimethicone cross polymer/cyclopentasiloxane/c yclohexasilicone | | | | |
| | Oil-phase component lauryl PEG-9 polydimethylsiloxyethyl dimethicone | 7.5 | 7.5 | 7.5 | 7.5 |
| | Oil-phase component distea admonium hectorite | 1.1 | 1.1 | 1.1 | 1.1 |
| | Antiseptic | 0.1 | 0.1 | 0.1 | 0.1 |
| | Oil-phase component-purified water | To 100 | To 100 | To 100 | To 100 |
| Aqueous-phase component | Butylene glycol | 5.0 | 5.0 | 5.0 | 5.0 |
| | Aqueous-phase component disodium EDTA | 0.05 | 0.05 | 0.05 | 0.05 |
| | Neutralizer | 0.1 | 0.1 | 0.1 | 0.1 |
| | Antiseptic | 0.1 | 0.1 | 0.1 | 0.1 |
| Powder component | EGCG-MOF-TiO₂ composite powder of Preparation Example 1 | 10 | - | - | - |
| | EGCG-MOF-ZnO composite powder of Preparation | - | 10 | - | - |
| | Example 2 | | | | |
| | GCG-MOF-TiO₂ composite powder of Preparation Example 3 | - | - | 10 | - |
| | Water-dispersion type of titanium dioxide powder | - | - | - | 10 |

### Preparation of Cosmetic Compositions: Examples 4 to 6 and Comparative Example 2

Cosmetic compositions of face powder formulation were prepared according to the compositions shown in Table 3 in a general method.

Specifically, Examples 4 to 6 and Comparative Example 2 were respectively prepared by mixing powder components according to the compositions shown in Table 3 with a hand mixer for 30 minutes, heating oil-phase components to uniformly melt the oil-phase components at 80 °C, spraying the oil-phased component melt to the powder components while the powder components were still being mixed, mixing them for 30 minutes, and then pulverizing the mixture with a pulverizer and filtering it to obtain a powder.

**(Table 3)**

| (unit: wt%) | | | | | |
|---|---|---|---|---|---|
| | Components | Ex. 4 | Ex. 5 | Ex. 6 | Comp. Ex. 2 |
| Oil-phase | Polyglyceryl-2 triisostearate | 4 | 4 | 4 | 4 |
| component | Oil-phase component dimethicone | 5 | 5 | 5 | 5 |
| | Oil-phase component polyoxyethylene hydrogenerated castor oil | 2 | 2 | 2 | 2 |
| | Antiseptic | 0.1 | 0.1 | 0.1 | 0.1 |
| Powder component | Talc (dimethicone treatment) | To 100 | To 100 | To 100 | To 100 |
| | Mica (dimethicone treatment) | 30 | 30 | 30 | 30 |
| | Sericite (dimethicone treatment) | 25 | 25 | 25 | 25 |
| | Silica | 5 | 5 | 5 | 5 |
| | EGCG-MOF-TiO₂ composite powder of Preparation Example 1 | 10 | - | - | - |
| | EGCG-MOF-ZnO composite powder of Preparation Example 2 | - | 10 | - | - |
| | GCG-MOF-TiO₂ composite powder of Preparation Example 3 | - | - | 10 | - |
| | General titanium dioxide powder | - | - | - | 10 |

### (Evaluation)

### Experimental Example 1: Confirmation of Surface Coating Film Formation (STEM)

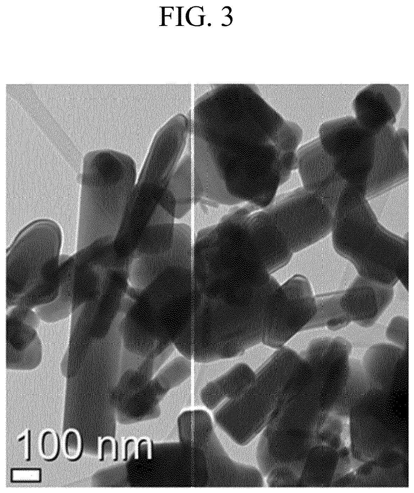

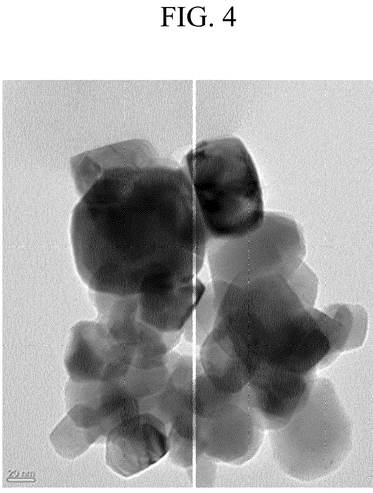

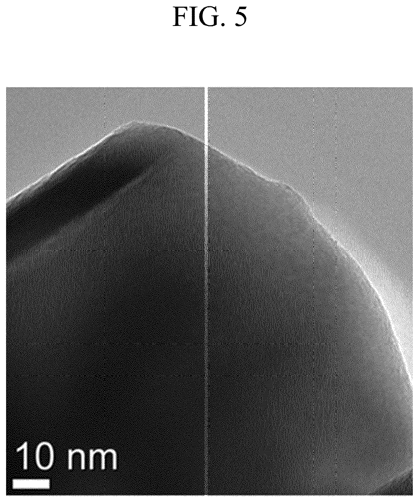

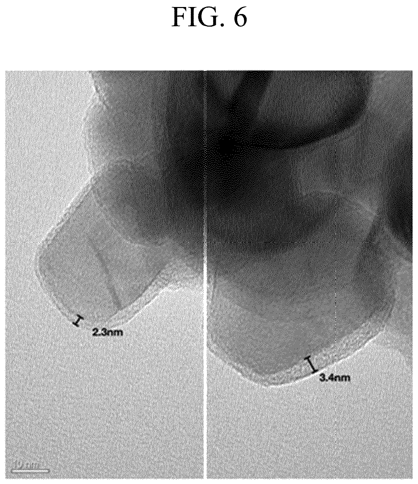

The surfaces of the surface-treated titanium dioxide nanoparticles according to Preparation Example 1 were examined with a scanning transmission electron microscope (Tecnai F20 G2), and the results are shown in FIGS. 3 to 6. Referring to FIGS. 3 to 6, unlike before the coating (surface-treatment), after the coating (surface-treatment), a uniform nano-thickness metal-organic framework (MOF) thin film was formed on the surfaces of the titanium dioxide nanoparticles.

### Experimental Example 2: Evaluation of Radical Removal Rate (Antioxidant Ability)

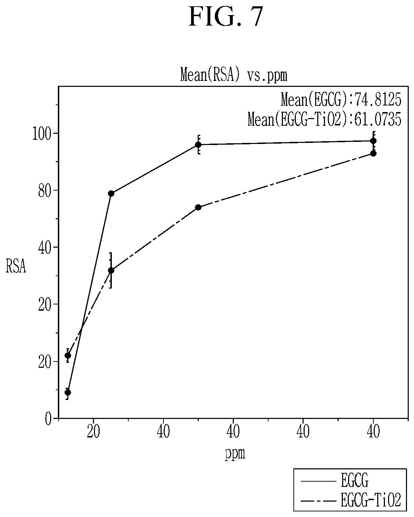

Antioxidant ability of EGCG bonded to the titanium dioxide nanoparticles according to Preparation Example 1 was evaluated in a DPPH test method. Even if EGCG is stabilized through formation of the metal-organic framework (MOF), its antioxidant ability may be lost, which may reduce the efficacy thereof, so the antioxidant ability was evaluated by measuring a radical removal rate, and the results are shown in FIG. 7. Referring to FIG. 7, the surface-treated titanium dioxide particles according to Preparation Example 1 exhibited a radical removal rate of about 70 % compared with an ECGC aqueous solution at the same concentration, and accordingly, even though EGCG was not supported in the pores of MOF but was formed into a MOF skeleton itself, the antioxidant ability was not lost.

### Experimental Example 3: Comparison of Color Stability Between Surface Treatment and Simple Mixing

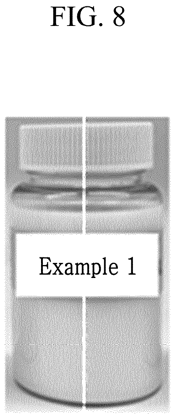

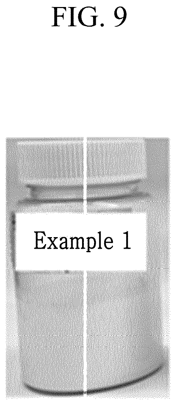

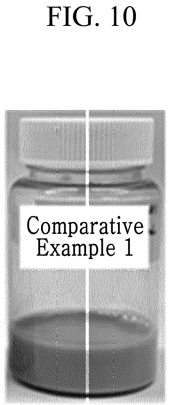

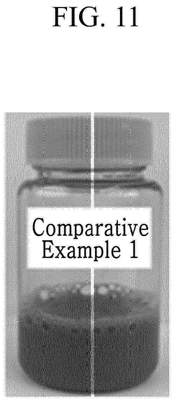

The cosmetic compositions of Example 1 and Comparative Example 1 were examined with respect to color changes with the naked eye (observed respectively at room temperature (25 °C) and a high temperature (40 °C) for 5 weeks), and the results are shown in Table 4 and FIGS. 8 to 11. Referring to Table 4 and FIGS. 8 to 11, the cosmetic composition according to Comparative Example 1 exhibited severe browning of EGCG, but the cosmetic composition according to Example 1 exhibited almost no browning of EGCG.

**(Table 4)**

| | Example 1 | Comparative Example 1 |
|---|---|---|
| Room temperature (25 °C) | ∘ (FIG. 8) | × (FIG. 10) |
| High temperature (40 °C) | ∘ (FIG. 9) | × (FIG. 11) |

| | | |
|---|---|---|
| ○: Even after 5 weeks, there was no visual color change compared with those 5 weeks ago. ×: After 5 weeks, there was a color change determined by the naked eye compared with 5 weeks previous (browning). | | |

### Experimental Example 4: Evaluation of Dispersion Stability

A surface potential of particles is strength of a repulsive force among the particles and is used as an index showing dispersion stability. Accordingly, how much a MOF coating thin film had an influence on the surface potential of titanium dioxide was evaluated by measuring a zeta potential, and the results are shown in Tables 5 and 6. Table 5 shows that surface potential was measured depending on pH changes on the surface of the composition (an aqueous solution) of Example 1, wherein the surface potential was increased as the pH was closer to a base, and Table 6 shows average surface potentials in the compositions (aqueous solutions) having pH 10 according to Comparative Example 1 and Examples 1, 2, and 3.

**(Table 5)**

| (unit: mV) | | | |
|---|---|---|---|
| | pH 4 | pH 7 | pH 10 |
| zeta potential | -4.81 | -26.94 | -37.82 |

**(Table 6)**

| (unit: mV) | | | | |
|---|---|---|---|---|
| | Comparative Example 1 | Example 1 | Example 2 | Example 3 |
| zeta potential | -26.43 | -37.82 | -41.52 | -45.37 |

Referring to Tables 5 and 6, surface potentials of titanium dioxide particles in the cosmetic compositions according to Examples 1 to 3 exhibited larger negative charge than that of titanium dioxide particles in the cosmetic composition according to Comparative Example 1, and in addition, Examples 1, 2, and 3 had a larger negative surface potential in order of Example 1 < Example 2 < Example 3. Accordingly, surface-treated inorganic particles according to an embodiment exhibits excellent dispersion stability compared with nonsurface-treated inorganic particles, and this dispersion stability may be controlled depending on a type of inorganic particles and a type of polyphenols forming a metal-organic framework.

## Claims

1. A surface-treated inorganic particle, including
inorganic particles and a metal-organic framework bound to the surface of the inorganic particles,
wherein catechins form a skeleton of the metal-organic framework.

2. The surface-treated inorganic particle of claim 1, wherein the catechins include epicatechin, epicatechin gallate, epigallocatechin, epigallocatechin gallate, catechin, catechin gallate, gallocatechin, gallocatechin gallate, or a combination thereof.

3. The surface-treated inorganic particle of claim 1 or 2, wherein the metal constituting the metal-organic framework includes iron, magnesium, zinc, copper, manganese, titanium, molybdenum, cerium, zirconium, barium, aluminum, calcium, yttrium, silver, gold, or a combination thereof.

4. The surface-treated inorganic particles of any one of claims 1 to 3, wherein the inorganic particles include titanium dioxide, zinc oxide, iron oxide, copper oxide, aluminum oxide, zirconium oxide, cerium oxide, barium oxide, silica, mica, talc, sericite, calamine, or a combination thereof.

5. The surface-treated inorganic particles of any one of claims 1 to 4, wherein the inorganic particles have a particle diameter of 10 nm to 100,000 nm.

6. A method of manufacturing the surface-treated inorganic particles of any one of claims 1 to 5, including:
coordinating catechins to the surface of inorganic particles;
polymerizing the coordinated catechins with metal ions; and
purifying the surface-treated inorganic particles.

7. The method of claim 6, wherein
the coordinating catechins to the surface of inorganic particles includes
adding the inorganic particles to an aqueous solution, dispersing the resultant, adding the catechins thereto, and stirring the resultant.

8. The method of claim 6 or 7, wherein
the polymerizing the coordinated catechins with metal ions includes
injecting metal ions or a cluster thereof to an aqueous solution in which inorganic particles having coordination bonds with catechins on the surface are dispersed.

9. The method of claim 7 or claim 8 when referring to claim 7, wherein a content of the catechins is less than or equal to 20 parts by weight relative to 100 parts by weight of the inorganic particles to be added.

10. The method of claim 7 or any one of claims 8 and 9 when referring to claim 7, wherein the adding of the inorganic particles to the aqueous solution, dispersing of the resultant, adding of the catechins thereto, and stirring of the resultant are performed for 1 minute to 60 minutes at a temperature of 10 °C to 30 °C under a pH condition of 4 to 9.

11. The method of claim 8 or any one of claims 9 and 10 when referring to claim 8, wherein the injecting of the metal ions or the cluster thereof to an aqueous solution in which inorganic particles having coordination bonds with catechins on the surface are dispersed includes stirring the solution for about 1 minute to 6 hours at 10 °C to 100 °C after injecting the metal ions or the cluster thereof.

12. The method of any one of claims 6 to 11, wherein the purifying of the surface-treated inorganic particles includes repeating the removal of a supernatant by filtration or centrifugation using a filter having nanopores or micropores.

13. The method of any one of claims 6 to 12, wherein the catechins include epicatechin, epicatechin gallate, epigallocatechin, epigallocatechin gallate, catechin, catechin gallate, gallocatechin, gallocatechin gallate, or a combination thereof, and/or
wherein the metal ion includes an iron ion, a magnesium ion, a zinc ion, a copper ion, a manganese ion, a titanium ion, a molybdenum ion, a cerium ion, a zirconium ion, a barium ion, an aluminum ion, a calcium ion, a yttrium ion, a silver ion, a gold ion, or a combination thereof, and/or
wherein the inorganic particles include titanium dioxide, zinc oxide, iron oxide, copper oxide, aluminum oxide, zirconium oxide, cerium oxide, barium oxide, silica, mica, talc, sericite, calamine, or a combination thereof, and/or
wherein the inorganic particles have a particle diameter of 10 nm to 100,000 nm.

14. A dispersion solution in which the surface-treated inorganic particles of any one of claims 1 to 5 are dispersed, wherein optionally a solid content in the dispersion solution is 0.1 wt% to 70 wt% relative to the total amount of the dispersion solution.

15. A cosmetic composition including
the surface-treated inorganic particles of any one of claims 1 to 5, or
the dispersion solution of claim 14.

## Patentansprüche

1. Oberflächenbehandeltes anorganisches Partikel, aufweisend:
anorganische Partikel und ein metallorganisches Gerüst, welches an die Oberfläche der anorganischen Partikel gebunden ist,
wobei Catechine ein Grundgerüst des metallorganischen Gerüsts bilden.

2. Oberflächenbehandeltes anorganisches Partikel gemäß Anspruch 1, wobei die Catechine aufweisen: Epicatechin, Epicatechingallat, Epigallocatechin, Epigallocatechingallat, Catechin, Catechingallat, Gallocatechin, Gallocatechingallat oder eine Kombination dieser.

3. Oberflächenbehandeltes anorganisches Partikel gemäß Anspruch 1 oder 2, wobei das Metall, welches das metallorganische Gerüst bildet, aufweist: Eisen, Magnesium, Zink, Kupfer, Mangan, Titan, Molybdän, Cer, Zirkonium, Barium, Aluminium, Calcium, Yttrium, Silber, Gold oder eine Kombination dieser.

4. Oberflächenbehandelte anorganische Partikel gemäß irgendeinem der Ansprüche 1 bis 3, wobei die anorganischen Partikel aufweisen: Titandioxid, Zinkoxid, Eisenoxid, Kupferoxid, Aluminiumoxid, Zirkoniumoxid, Ceroxid, Bariumoxid, Siliciumdioxid, Glimmer, Talk, Serizit, Kalamin oder eine Kombination dieser.

5. Oberflächenbehandelte anorganische Partikel gemäß irgendeinem der Ansprüche 1 bis 4, wobei die anorganischen Partikel einen Partikeldurchmesser von 10 nm bis 100.000 nm aufweisen.

6. Verfahren zur Herstellung der oberflächenbehandelten anorganischen Partikel gemäß irgendeinem der Ansprüche 1 bis 5, aufweisend:
Koordinieren von Catechinen an die Oberfläche der anorganischen Partikel;
Polymerisieren der koordinierten Catechine mit Metallionen; und
Aufreinigen der oberflächenbehandelten anorganischen Partikel.

7. Verfahren gemäß Anspruch 6, wobei
das Koordinieren der Catechine an die Oberfläche der anorganischen Partikel aufweist:
Hinzufügen der anorganischen Partikel zu einer wässrigen Lösung, Dispergieren des Erhaltenen, Hinzufügen der Catechine, und Rühren des Erhaltenen.

8. Verfahren gemäß Anspruch 6 oder 7, wobei das Polymerisieren der koordinierten Catechine mit Metallionen aufweist:
Injizieren von Metallionen, oder eines Clusters dieser, zu einer wässrigen Lösung, in der anorganische Partikel mit Koordinationsbindungen zu Catechinen auf der Oberfläche dispergiert sind.

9. Verfahren gemäß Anspruch 7 oder Anspruch 8, rückbezogen auf Anspruch 7, wobei der Gehalt an Catechinen weniger oder gleich 20 Gewichtsteile beträgt, bezogen auf 100 Gewichtsteile der zuzusetzenden anorganischen Partikel.

10. Verfahren gemäß Anspruch 7 oder irgendeinem der Ansprüche 8 und 9, rückbezogen auf Anspruch 7, wobei das Hinzufügen der anorganischen Partikel zu der wässrigen Lösung, Dispergieren des Erhaltenen, Hinzufügen der Catechine, und Rühren des Erhaltenen für 1 Minute bis 60 Minuten durchgeführt werden, bei einer Temperatur von 10 °C bis 30 °C, unter einer pH-Bedingung von 4 bis 9.

11. Verfahren nach Anspruch 8 oder irgendeinem der Ansprüche 9 und 10, rückbezogen auf Anspruch 8, wobei das Injizieren der Metallionen oder deren Cluster in eine wässrige Lösung, in der anorganische Partikel mit Koordinationsbindungen zu Catechinen auf der Oberfläche dispergiert sind, Rühren der Lösung für 1 Minute bis 6 Stunden aufweist, bei 10 °C bis 100 °C, nach Injizieren der Metallionen oder deren Cluster.

12. Verfahren gemäß irgendeinem der Ansprüche 6 bis 11, wobei das Aufreinigen der oberflächenbehandelten anorganischen Partikel aufweist: das Wiederholen des Entfernens eines Überstands durch Filtration oder Zentrifugation, unter Verwendung eines Filters mit Nanoporen oder Mikroporen.

13. Verfahren gemäß einem der Ansprüche 6 bis 12,
wobei die Catechine aufweisen: Epicatechin, Epicatechingallat, Epigallocatechin, Epigallocatechingallat, Catechin, Catechingallat, Gallocatechin, Gallocatechingallat oder eine Kombination dieser, und/oder
wobei das Metallion aufweist: ein Eisenion, ein Magnesiumion, ein Zinkion, ein Kupferion, ein Manganion, ein Titanion, ein Molybdänion, ein Cerion, ein Zirkoniumion, ein Bariumion, ein Aluminiumion, ein Calciumion, ein Yttriumion, ein Silberion, ein Goldion oder eine Kombination dieser, und/oder
wobei die anorganischen Partikel aufweisen: Titandioxid, Zinkoxid, Eisenoxid, Kupferoxid, Aluminiumoxid, Zirkoniumoxid, Ceroxid, Bariumoxid, Siliciumdioxid, Glimmer, Talk, Serizit, Kalamin oder eine Kombination dieser, und/oder
wobei die anorganischen Partikel einen Partikeldurchmesser von 10 nm bis 100.000 nm aufweisen.

14. Dispersionslösung, in der die oberflächenbehandelten, anorganischen Partikel gemäß irgendeinem der Ansprüche 1 bis 5 dispergiert sind, wobei optional ein Feststoffgehalt in der Dispersionslösung 0,1 Gew.-% bis 70 Gew.-% beträgt, bezogen auf die Gesamtmenge der Dispersionslösung.

15. Kosmetische Zusammensetzung, aufweisend:
die oberflächenbehandelten anorganischen Partikel gemäß irgendeinem der Ansprüche 1 bis 5, oder
die Dispersionslösung gemäß Anspruch 14.

## Revendications

1. Particule inorganique traitée en surface, comprenant :
des particules inorganiques et une structure organométallique liée à la surface des particules inorganiques,
dans laquelle des catéchines forment un squelette de la structure organométallique.

2. Particule inorganique traitée en surface selon la revendication 1, dans laquelle les catéchines comprennent l'épicatéchine, le gallate d'épicatéchine, l'épigallocatéchine, le gallate d'épigallocatéchine, la catéchine, le gallate de catéchine, la gallocatéchine, le gallate de gallocatéchine, ou une combinaison de ceux-ci.

3. Particule inorganique traitée en surface selon la revendication 1 ou 2, dans laquelle le métal constituant la structure organométallique comprend du fer, du magnésium, du zinc, du cuivre, du manganèse, du titane, du molybdène, du cérium, du zirconium, du baryum, de l'aluminium, du calcium, de l'yttrium, de l'argent, de l'or ou une combinaison de ceux-ci.

4. Les particules inorganiques traitées en surface selon l'une quelconque des revendications 1 à 3, dans lesquelles les particules inorganiques comprennent du dioxyde de titane, de l'oxyde de zinc, de l'oxyde de fer, de l'oxyde de cuivre, de l'oxyde d'aluminium, de l'oxyde de zirconium, de l'oxyde de cérium, de l'oxyde de baryum, de la silice, du mica, du talc, de la séricite, de la calamine, ou une combinaison de ceux-ci.

5. Les particules inorganiques traitées en surface selon l'une quelconque des revendications 1 à 4, dans lesquelles les particules inorganiques ont un diamètre de particule de 10 nm à 100 000 nm.

6. Procédé de fabrication des particules inorganiques traitées en surface selon l'une quelconque des revendications 1 à 5, comprenant :
la coordination de catéchines à la surface de particules inorganiques ;
la polymérisation des catéchines coordonnées avec des ions métalliques ; et
la purification des particules inorganiques traitées en surface.

7. Procédé selon la revendication 6, dans lequel
la coordination des catéchines à la surface de particules inorganiques comprend l'ajout des particules inorganiques à une solution aqueuse, la dispersion du résultat, l'ajout des catéchines à celui-ci et l'agitation du résultat.

8. Procédé selon la revendication 6 ou 7, dans lequel
la polymérisation des catéchines coordonnées avec des ions métalliques comprend
l'injection d'ions métalliques ou d'un cluster de ceux-ci dans une solution aqueuse dans laquelle sont dispersées des particules inorganiques ayant des liaisons de coordination avec des catéchines à la surface.

9. Procédé selon la revendication 7 ou la revendication 8 en référence à la revendication 7,
dans lequel une teneur en catéchines est inférieure ou égale à 20 parties en poids par rapport à 100 parties en poids des particules inorganiques à ajouter.

10. Procédé selon la revendication 7 ou l'une quelconque des revendications 8 et 9 en référence à la revendication 7, dans lequel l'ajout des particules inorganiques à la solution aqueuse, la dispersion du résultat, l'ajout des catéchines à celui-ci et l'agitation du résultat sont effectués pendant 1 minute à 60 minutes à une température de 10 °C à 30 °C dans des conditions de pH de 4 à 9.

11. Procédé selon la revendication 8 ou l'une quelconque des revendications 9 et 10 en référence à la revendication 8, dans lequel l'injection des ions métalliques ou de leur cluster dans une solution aqueuse dans laquelle sont dispersées des particules inorganiques ayant des liaisons de coordination avec des catéchines à la surface comprend l'agitation de la solution pendant environ 1 minute à 6 heures à 10 °C à 100 °C après l'injection des ions métalliques ou de leur cluster.

12. Procédé selon l'une quelconque des revendications 6 à 11, dans lequel la purification des particules inorganiques traitées en surface comprend la répétition de l'élimination d'un surnageant par filtration ou centrifugation en utilisant un filtre comportant des nanopores ou des micropores.

13. Procédé selon l'une quelconque des revendications 6 à 12, dans lequel les catéchines comprennent l'épicatéchine, le gallate d'épicatéchine, l'épigallocatéchine, le gallate d'épigallocatéchine, la catéchine, le gallate de catéchine, la gallocatéchine, le gallate de gallocatéchine, ou une combinaison de ceux-ci, et/ou
dans lequel l'ion métallique comprend un ion fer, un ion magnésium, un ion zinc, un ion cuivre, un ion manganèse, un ion titane, un ion molybdène, un ion cérium, un ion zirconium, un ion baryum, un ion aluminium, un ion calcium, un ion yttrium, un ion argent, un ion or, ou une combinaison de ceux-ci, et/ou
dans lequel les particules inorganiques comprennent du dioxyde de titane, de l'oxyde de zinc, de l'oxyde de fer, de l'oxyde de cuivre, de l'oxyde d'aluminium, de l'oxyde de zirconium, de l'oxyde de cérium, de l'oxyde de baryum, de la silice, du mica, du talc, de la séricite, de la calamine, ou une combinaison de ceux-ci, et/ou
dans lequel les particules inorganiques ont un diamètre de particule de 10 nm à 100 000 nm.

14. Solution de dispersion dans laquelle sont dispersées les particules inorganiques traitées en surface selon l'une quelconque des revendications 1 à 5, dans laquelle, éventuellement, une teneur en solides dans la solution de dispersion est de 0,1 % en poids à 70 % en poids par rapport à la quantité totale de la solution de dispersion.

15. Composition cosmétique, comprenant :
les particules inorganiques traitées en surface selon l'une quelconque des revendications 1 à 5, ou
la solution de dispersion selon la revendication 14.
